# EUROPÄISCHE PATENTSCHRIFT

(11) **EP 3 422 002 B1**
(45) Veröffentlichungstag und Bekanntmachung des Hinweises auf die Patenterteilung: **08.04.2020**
(21) Anmeldenummer: 17177870.7
(22) Anmeldetag: 26.06.2017
(51) Int. Cl.: G01N 33/569, G01N 33/574, G01N 33/72

(54) **SCREENINGVERFAHREN ZUR DIAGNOSE EINER HÄMATOLOGISCHEN NEOPLASIE**
SCREENING METHOD FOR DIAGNOSIS OF HEMATOLOGIC NEOPLASIA
PROCÉDÉ DE DÉPISTAGE DESTINÉ AU DIAGNOSTIC D'UN NÉOPLASME HÉMATOLOGIQUE

(43) Veröffentlichungstag der Anmeldung: 02.01.2019
(73) Patentinhaber: Bioscentia Institut für Medizinische Diagnostik GmbH, 55218 Ingelheim (DE)
(72) Erfinder: KAMINSKI, Wolfgang Prof. Dr., 55218 Ingelheim (DE)
(74) Vertreter: Cohausz & Florack

(56) Entgegenhaltungen:
- WO-A1-2015/119570
- WO-A1-2016/196580
- WO-A2-2013/009690
- WO-A2-2016/144728
- US-A1- 2010 009 364
- D. OSCIER ET AL: "Guidelines on the diagnosis and management of chronic lymphocytic leukaemia", BRITISH JOURNAL OF HAEMATOLOGY, Bd. 125, Nr. 3, 1. Mai 2004 (2004-05-01), Seiten 294-317, XP055429447, GB ISSN: 0007-1048, DOI: 10.1111/j.1365-2141.2004.04898.x
- F. E. CRAIG: "The utility of peripheral blood smear review for identifying specimens for flow cytometric immunophenotyping", INTERNATIONAL JOURNAL OF LABORATORY HEMATOLOGY, Bd. 39, 26. April 2017 (2017-04-26), Seiten 41-46, XP055429688, GB; US ISSN: 1751-5521, DOI: 10.1111/ijlh.12651
- JAN CERNY ET AL: "Why Does My Patient Have Leukocytosis?", HEMATOLOGY - ONCOLOGY CLINICS OF NORTH AMERICA, Bd. 26, Nr. 2, 1. April 2012 (2012-04-01), Seiten 303-319, XP055429650, US ISSN: 0889-8588, DOI: 10.1016/j.hoc.2012.01.001
- DAVID J FORAN ET AL: "Computer-Assisted Discrimination Among Malignant Lymphomas and Leukemia Using Immunophenotyping, Intelligent Image Repositories, and Telemicroscopy", IEEE TRANSACTIONS ON INFORMATION TECHNOLOGY IN BIOMEDICINE, IEEE SERVICE CENTER, LOS ALAMITOS, CA, US, Bd. 4, Nr. 4, 1. Dezember 2000 (2000-12-01), XP011028213, ISSN: 1089-7771
- WANG ENDI ET AL: "An Epstein-Barr virus-positive diffuse large B-cell lymphoma presenting as multi-organ failure: A catastrophic lymphomatosis with fulminant visceral organ dissemination resulting in a precipitous death in a 59-year-old female with no identifiable etiology for immunodefici", PATHOLOGY - RESEARCH AND PRACTICE, ELSEVIER, AMSTERDAM, NL, Bd. 210, Nr. 1, 17. September 2013 (2013-09-17), Seiten 62-66, XP028548092, ISSN: 0344-0338, DOI: 10.1016/J.PRP.2013.09.001

## Beschreibung

Die vorliegende Erfindung betrifft ein Screeningverfahren zur Diagnose einer hämatologischen Neoplasie. Weiterhin betrifft die Erfindung auch ein System, ein Computerprogrammprodukt und ein Speichermedium.

Aus dem Stand der Technik ist es bekannt, Erkrankungen oder potentielle Erkrankungen mittels eines Screeningverfahrens von Blut im Labor zu diagnostizieren. Auch im Bereich der Krebserkrankungen, wie etwa bei Prostata- oder Darmkrebs, ist es bekannt, Screeningverfahren durchzuführen, die eine systematische Früherkennung der Krankheit erlauben.

Ein derartiges Verfahren existiert im Bereich von Krebs des blutbildenden oder lymphatischen Systems (im Folgenden auch kurz "Blutkrebs") jedoch nicht, obwohl beispielsweise in Deutschland rund 35.000 Individuen jeder Altersgruppe jährlich hieran erkranken. Dabei wird im Wesentlichen zwischen den zwei Hauptgruppen "Leukämien" und "Lymphomen" und insgesamt nach aktuellem Stand zwischen 173 verschiedenen Blutkrebsarten unterschieden. Problematisch ist, dass die Krankheit zunächst unbemerkt verläuft und erst im fortgeschrittenen Stadium zu fassbaren Krankheitssymptomen führt, die sich klinisch manifestieren. Erschwerend kommt weiterhin hinzu, dass selbst dann die verursachten Symptome und Laborbefunde unspezifisch sind und von denen weitaus häufigere Erkrankungen kaum zu unterscheiden sind. Die hohe Anzahl an Blutkrebsarten und die fehlenden bzw. unspezifischen Symptome führen dazu, dass bisher kein zuverlässiges Screeningverfahren zur Erkennung von Blutkrebs existiert. Im Ergebnis bleibt der Blutkrebs meist lange unerkannt und wird erst sehr spät und damit im fortgeschrittenen Stadium vom Arzt erkannt. Frühzeitige und damit möglicherweise lebensrettende Therapien sind bei dieser Krebsart damit bisher nicht zuverlässig zu realisieren. Vielmehr ist die Diagnose von Blutkrebs in der derzeit gängigen Praxis nicht selten ein Zufallsbefund, zumeist auf Basis eines drastisch auffälligen Laborbefundes, wie er typischerweise erst im weit fortgeschrittenen Stadium vorkommt. Die Diagnosefindung von Blutkrebs ist somit nach dem derzeitigen Stand der Technik kompliziert, zeitaufwendig, kostenträchtig und erfordert ausgewiesenes Expertenwissen.

US2010/0009364 beschreibt ein Verfahren zur Diagnose akuter myeloischer Leukämie. Aus einer flüssigen Blutprobe wird ein großes Blutbild bestimmt. Abweichungen in dabei erfassten Werten für weiße Blutzellen, Hämoglobin, Hämatokrit, Thrombozyten und zirkulierende Blasten führen zu einer Bestätigung der Diagnose in einer Knochenmarkprobe, wobei in dieser der Anteil unreifer Blasten analysiert wird mittels Immunphänotpyisierung bzw. Durchflusszytometrie.

WO2015/119570 offenbart die Diagnose akuter myeloischer Leukämie anhand einer abweichenden Zahl weißer Blutzellen im großen Blutbild. Zur vorläufigen Diagnose wird ein Blutausstrich hinsichtlich leukämischer Blasten, untersucht. Zur abschließenden Klärung wird Knochenmarks mikroskopisch oder per Durchflusszytometrie untersucht.

Oscier und Mitarbeiter (Br J Hematol. (2004) 125:294-317) beschreiben diagnostische Verfahren zur Diagnose chronischer lymphatischer Leukämie, wobei ein routinemäßig erstelltes großes Blutbild anhand der Lymphozytenzahl und der charakteristischen Lymphozytenmorphologie analysiert wird. Zur weiteren Klärung wird der Immunphänotyp oder ein weiteres Blutbild herangezogen.

Craig und Mitarbeiter (Int J Lab Hematol. 2017) 39:41-46) verwenden zur Diagnose hematolymphoider Neoplasien Abweichungen der Lymphozyten im großen Blutbild in Kombination mit einem manuell durchgeführten Blutausstrich, um verdächtige Proben weiter per durchflusszytometrischer Immunphänotypisierung zu untersuchen Cerny und Mitarbeiter (Hematol Oncol Clin N Am (2012) 26:303-319) evaluieren Leukozytose als Ausgangspunkt zur Diagnose von Leukämie oder myeloproliferativen Neoplasmen mittels Zellbestimmung im großen Blutbildes und eines Blutausstrichs, wobei eine abschließende Klärung mittels Knochenmarksuntersuchung erfolgt.

WO 2016/144728 beschreibt die Diagnose von AML mittels großem Blutbild, wobei Abweichungen in der Zahl weißer Blutzellen, Anwesenheit leukämischer Blasten, verringerte Zahl von Thrombozyten und roten Blutzellen analysiert werden. Eine vorläufige Diagnose kann auf Basis leukämischer Blasten im Blutausstrich erfolgen, die Bestätigung mittels Knochenmarkuntersuchung.

Systematische Screeningverfahren für Blutkrebs an Proben von großen Patientenkollektiven, wie sie beispielswiese in einem Großlabor täglich anfallen, waren aus diesen Gründen bisher nicht realisierbar. Es war insbesondere bisher nicht gelungen, ein Verfahren mit Kriterien aufzustellen, mit welchem Proben von gesunden und kranken Personen in Bezug auf hämatologische Neoplasien entsprechend identifiziert werden konnten, also insbesondere falsch positive und falsch negative Diagnosen auf ein Minimum reduziert bzw. vermieden wurden.

Vor diesem Hintergrund besteht nun die Aufgabe, ein Screeningverfahren anzugeben, mit welchem effizient und effektiv, das heißt insbesondere kostengünstig, schnell und zuverlässig, die Diagnose einer (potentiellen) hämatologischen Neoplasie erlaubt.

Die Aufgabe wird gemäß einem ersten Aspekt der Erfindung durch ein Screeningverfahren zur Diagnose einer hämatologischen Neoplasie gelöst, umfassend die Schritte:
- Durchführen einer ersten Prüfstufe zur Prüfung, ob eine potentielle hämatologische Neoplasie vorliegt, wobei die erste Prüfstufe teilweise oder vollständig automatisiert durchgeführt wird, wobei im Rahmen der ersten Prüfstufe zumindest ein erstes Hämatogramm mit einer Blutprobe in der Flüssigphase erstellt wird und auf Basis eines ersten Satzes von Filterregeln geprüft wird, ob das erste Hämatogramm von einem gemäß dem ersten Satz von Filterregeln vorgegebenen Sollzustand abweicht, wobei der erste Satz von Filterregeln zumindest zwei Filterregeln umfasst, wobei das zumindest erste Hämatogramm einen Hämoglobinwert und einen Thrombozytenwert umfasst, und wobei eine Filterregel des ersten Satzes von Filterregeln der ersten Prüfstufe auf dem Hämoglobinwert und dem Thrombozytenwert basiert, und
- Durchführen einer zweiten Prüfstufe zur Prüfung, ob eine potentielle hämatologische Neoplasie vorliegt, wenn die Prüfung der ersten Prüfstufe ergibt, dass eine potentielle hämatologische Neoplasie vorliegt, wobei die zweite Prüfstufe teilweise oder vollständig automatisiert durchgeführt wird, wobei im Rahmen der zweiten Prüfstufe ein morphologisches Analyseergebnis einer Festphase der Blutprobe ermittelt wird und auf Basis eines zweiten Satzes von Filterregeln geprüft wird, ob das morphologische Analyseergebnis der zweiten Prüfstufe von einem gemäß dem zweiten Satz von Filterregeln vorgegebenen Sollzustand abweicht, und wobei der zweite Satz von Filterregeln zumindest zwei Filterregeln umfasst.

Gemäß der Erfindung wurde zunächst erkannt, dass die Diagnose einer hämatologischen Neoplasie dadurch einem effektiven und effizienten systematischen Screeningverfahren zugänglich wird, indem das Verfahren zumindest in eine erste und eine zweite Prüfstufe aufgeteilt wird. In jeder Prüfstufe wird dabei geprüft, ob eine potentielle hämatologische Neoplasie vorliegt. Ergibt bereits die erste Prüfstufe, dass keine hämatologische Neoplasie vorliegt, braucht die zweite Prüfstufe beispielsweise nicht mehr durchgeführt zu werden. Dabei umfasst jedenfalls die erste Prüfstufe die Erstellung eines ersten Hämatogramms mit einer Blutprobe. Die Erfindung schlägt nun vor, auf Basis eines ersten Satzes von Filterregeln zu prüfen, ob das erste Hämatogramm von einem Sollzustand abweicht, der gemäß dem ersten Satz von Filterregeln vorgegebenen wird. Es hat sich gezeigt, dass durch die Definition eines Satzes von Filterregeln im Rahmen einer ersten Prüfstufe, die einer zweiten Prüfstufe vorgelagert ist, eine effiziente und effektiven Prüfung eines Vorliegens einer potentiellen hämatologischen Neoplasie ermöglicht wird.

Es hat sich insbesondere gezeigt, dass es dadurch sogar möglich ist, die Anzahl von Blutproben, welche der zweiten Prüfstufe unterzogen werden müssen, auf unter 1% der Anzahl der Blutproben, welche noch die erste Prüfstufe durchlaufen, zu reduzieren. Durch die zweite Prüfstufe kann zudem die Anzahl der Blutproben, welche möglichen weiteren Untersuchungen unterzogen werden müssen, weiterhin auf etwa 2% der Blutproben, welche die zweite Prüfstufe durchlaufen, reduziert werden. Auf diese Weise wird die Diagnose einer hämatologischen Neoplasie im Rahmen eines Massenscreenings realisierbar.

Das Verfahren kann somit insbesondere als ein systematisches Verfahren zum mehrstufigen (Massen)-Screening verstanden werden, welches zumindest eine erste und eine zweite Prüfstufe umfasst. Es ist dabei jedoch nicht ausgeschlossenen, dass das Verfahren weitere vorgelagerte, nachgelagerte und/oder zwischengelagerte Prüfstufen umfassen kann.

Unter hämatologischen Neoplasien werden insbesondere maligne Neoplasien verstanden wie sie bei Leukämien oder Lymphomen auftreten.

Die Blutprobe ist vorzugsweise eine (EDTA-)Vollblutprobe. Das erste Hämatogramm wird auf Basis einer Blutprobe in der Flüssigphase erstellt. Das erste Hämatogramm kann insbesondere ein kleines oder großes Blutbild, vorzugsweise jedoch ein kleines Blutbild, sein. Es hat sich gezeigt, dass die hierdurch erreichte Genauigkeit für die durchzuführende Prüfung ausreichend ist, was wiederum eine besonders hohe Effizienz hinsichtlich des Zeit- und Kostenaufwandes ermöglicht. Im Rahmen der ersten Prüfstufe kann insbesondere auch noch ein weiteres Hämatogramm erstellt werden, wie im Folgenden noch näher erläutert wird.

In einem Beispiel umfasst die erste Prüfstufe lediglich die Erstellung eines ersten Hämatogramms. In diesem Fall wird vorzugsweise dann entschieden, dass gemäß der ersten Prüfstufe eine potentielle hämatologische Neoplasie vorliegt, wenn das erste Hämatogramm von dem gemäß dem ersten Satz von Filterregeln vorgegebenen Sollzustand abweicht. Umfasst die erste Prüfstufe beispielsweise zudem die Erstellung eines zweiten Hämatogramms, wird in diesem Fall vorzugsweise erst entschieden, dass gemäß der ersten Prüfstufe eine potentielle hämatologische Neoplasie vorliegt, wenn das zweite Hämatogramm von dem gemäß dem ersten Satz von Filterregeln vorgegebenen Sollzustand abweicht. Dass das erste Hämatogramm von dem gemäß dem ersten Satz von Filterregeln vorgegebenen Sollzustand abweicht, führt in diesem Fall beispielsweise lediglich dazu, dass zunächst das zweite Hämatogramm erstellt wird, was im Folgenden auch noch genauer beschrieben wird.

Vorzugsweise gibt der Satz von Filterregeln einen Sollzustand vor, während zudem auch jede Filterregel des Satzes von Filterregeln für sich einen jeweiligen Sollzustand vorgibt. So weicht das erste Hämatogramm von einem gemäß dem ersten Satz von Filterregeln vorgegebenen Sollzustand beispielsweise dann ab, wenn das erste Hämatogramm beispielsweise gemäß lediglich einer einzigen Filterregel, gemäß mehrerer Filterregeln oder aber erst gemäß aller Filterregeln des ersten Satzes von Filterregeln der ersten Prüfstufe vom durch die jeweilige(n) Filterregel(n) vorgegebenen Sollzustand abweicht. Wie im Folgenden noch beschrieben wird, ist es allerdings bevorzugt, wenn eine Abweichung bereits gemäß einer einzigen Filterregel des ersten Satzes von Filterregeln ausreicht, um festzustellen, dass das erste Hämatogramm von einem gemäß dem ersten Satz von Filterregeln vorgegebenen Sollzustand abweicht.

Durch das Hämatogramm werden insbesondere eine oder mehrere Blutwerte bestimmt. Eine Filterregel gibt beispielsweise einen Sollzustand insbesondere hinsichtlich eines oder mehrerer der im Rahmen des Hämatogramms ermittelten Blutwerte vor. Ein Blutwert kann insbesondere eine Information zur Quantität (beispielsweise eine relative oder absolute Menge) eines (zellulären) Blutbestandteils sein.

Die erste Prüfstufe wird teilweise oder vollständig automatisiert durchgeführt werden, beispielsweise durch ein System gemäß dem zweiten Aspekt. Insbesondere zur Erstellung und/oder Prüfung des ersten Hämatogramms kann ein erster automatischer Zellenanalysator vorgesehen sein.

Der erste Satz von Filterregeln umfasst bevorzugt mehrere Filterregeln. Erfindungsgemäß umfasst der erste Satz von Filterregeln zumindest zwei Filterregeln. Vorzugsweise umfasst der erste Satz von Filterregeln höchstens zehn, vorzugsweise höchstens fünf Filterregeln. Besonders bevorzugt umfasst der erste Satz von Filterregeln genau vier Filterregeln. Jede Filterregel kann dabei beispielsweise auf einem oder mehreren Blutwerten des Hämatogramms basieren.

Gemäß einer bevorzugten Ausgestaltung des Screeningverfahrens gemäß dem ersten Aspekt wird im Rahmen der ersten Prüfstufe, wenn das erste Hämatogramm von dem gemäß dem ersten Satz von Filterregeln vorgegebenen Sollzustand abweicht, mit der Blutprobe weiterhin ein zweites Hämatogramm erstellt, welches ein höhere Genauigkeit aufweist als das erste Hämatogramm, und auf Basis des ersten Satzes von Filterregeln geprüft, ob das zweite Hämatogramm von dem gemäß dem ersten Satz von Filterregeln vorgegebenen Sollzustand abweicht. Es hat sich gezeigt, dass hierdurch die Effizienz des Verfahrens bei hoher Effektivität weiterhin gesteigert werden kann. Besonders vorteilhaft ist in diesem Fall das erste Hämatogramm ein kleines Blutbild, während das zweite Hämatogramm ein großes Blutbild ist. Zur Erstellung und/oder Prüfung des zweiten Hämatogramms kann ebenfalls ein zweiter automatischer Zellenanalysator (mit einer höheren Genauigkeit als der erste) vorgesehen sein.

Wie bereits erwähnt, wird bei dieser Ausgestaltung das zweite Hämatogramm erstellt, wenn das erste Hämatogramm von dem gemäß dem ersten Satz von Filterregeln vorgegebenen Sollzustand abweicht. Weicht dann das zweite Hämatogramm von dem gemäß dem ersten Satz von Filterregeln vorgegebenen Sollzustand ab, wird vorzugsweise entschieden, dass gemäß der ersten Prüfstufe eine potentielle hämatologische Neoplasie vorliegt. In dieser Hinsicht gelten die Ausführungen in Bezug auf das erste Hämatogramm. So weicht insbesondere auch das zweite Hämatogramm von einem gemäß dem ersten Satz von Filterregeln vorgegebenen Sollzustand bevorzugt bereits dann ab, wenn das zweite Hämatogramm beispielsweise gemäß lediglich einer einzigen Filterregel des ersten Satzes von Filterregeln der ersten Prüfstufe vom durch die jeweilige Filterregel vorgegebenen Sollzustand abweicht.

Sowohl das erste Hämatogramm als auch das zweite Hämatogramm werden in diesem Fall jeweils auf Basis des ersten Satzes von Filterregeln geprüft. Grundsätzlich wäre jedoch auch vorstellbar, dass für das erste und das zweite Hämatogramm unterschiedliche Sätze von Filterregeln verwendet werden.

Gemäß einer bevorzugten Ausgestaltung des Screeningverfahrens gemäß dem ersten Aspekt weicht das zumindest erste Hämatogramm (und gegebenenfalls das zweite Hämatogramm) von dem gemäß dem ersten Satz von Filterregeln vorgegebenen Sollzustand bereits dann ab, wenn das zumindest erste Hämatogramm von einem gemäß einer Filterregel des ersten Satzes von Filterregeln vorgegebenen Sollzustand abweicht. Es hat sich gezeigt, dass es im Rahmen der ersten Prüfstufe bei einem Satz von Filterregeln vorteilhaft in Bezug auf ein effektives und effizientes Screening ist, bereits dann eine Abweichung des ersten und optional zweiten Hämatogramms vom durch den gesamten Satz von Filterregeln vorgegebenen Sollzustand anzunehmen, wenn dies gemäß einer Filterregel der Fall ist.

Gemäß einem Beispiel des Screeningverfahrens umfasst das zumindest erste Hämatogramm (und gegebenenfalls das zweite Hämatogramm) einen ,abnormale Lymphozyten'-Wert, einen ,unreife Granulozyten'-Wert und/oder einen Blasten-Wert, wobei eine Filterregel des ersten Satzes von Filterregeln der ersten Prüfstufe auf dem ,abnormale Lymphozyten'-Wert, dem "unreife Granulozyten"-Wert und/oder dem Blastenwert basiert. Es hat sich gezeigt, dass ein erster Satz von Filterregeln, welcher eine auf dem ,abnormale Lymphozyten'-Wert, dem ,unreife Granulozyten'-Wert und/oder dem Blastenwert basierende Filterregel umfasst, vorteilhaft hinsichtlich einer hohen Effektivität und Effizienz der ersten Prüfstufe ist. Diese Filterregel kann beispielsweise als erste Filterregel bezeichnet werden. Die jeweiligen Blutwerte können insbesondere eine Information zur Quantität (beispielsweise eine Präsenz oder eine relative oder absolute Menge) des jeweiligen Blutbestandteils umfassen. Vorzugsweise basiert die Filterregel auf dem ,abnormale Lymphozyten'-Wert, auf dem ,unreife Granulozyten'-Wert und dem Blastenwert, als auf allen drei genannten Blutwerten. Vorzugsweise weicht dabei zumindest das erste Hämatogramm vom gemäß dieser Filterregel vorgegebenen Sollzustand ab, wenn eine Präsenz entweder von abnormalen Lymphozyten oder von unreifen Ganulozyten oder von Blasten gegeben ist. Gleiches gilt für ein gegebenenfalls zweites Hämatogramm der ersten Prüfstufe.

Gemäß einem Beispiel des Screeningverfahrens umfasst das zumindest erste Hämatogramm (und gegebenenfalls das zweite Hämatogramm) einen Leukozytenwert, wobei eine Filterregel des ersten Satzes von Filterregeln der ersten Prüfstufe auf dem Leukozytenwert basiert. Es hat sich gezeigt, dass ein erster Satz von Filterregeln, welcher eine auf dem Leukozytenwert basierende Filterregel umfasst, vorteilhaft hinsichtlich einer hohen Effektivität und Effizienz der ersten Prüfstufe ist. Diese Filterregel kann beispielsweise als zweite Filterregel bezeichnet werden. Der Leukozytenwert kann insbesondere eine Information zur Quantität (beispielsweise eine relative oder absolute Menge, beispielsweise Anzahl/Volumen) der Leukozyten umfassen. Gleiches gilt für ein gegebenenfalls zweites Hämatogramm der ersten Prüfstufe.

Gemäß einem Beispiel des Screeningverfahrens weicht das zumindest erste Hämatogramm (und gegebenenfalls das zweite Hämatogramm) vom gemäß der auf dem Leukozytenwert basierenden Filterregel (zweite Filterregel) der ersten Prüfstufe vorgegebenen Sollzustand ab, wenn der Leukozytenwert über einem Schwellwert liegt oder diesem entspricht. Unter dem Leukozytenwert wird dabei insbesondere ein Gesamtwert hinsichtlich aller Leukozyten verstanden. Vorzugsweise ist der Schwellwert größer als 40.000, vorzugsweise größer al 45.000 Leukozyten/µL. Vorzugsweise ist der Schwellwert geringer als 200.000, vorzugsweise geringer als 100.000 Leukozyten/µL. Vorzugsweise liegt der Schwellwert etwa bei 50.000 Leukozyten/µL.

Gemäß dem erfindungsgemässen Screeningverfahren umfasst der erste Satz von Filterregeln zumindest zwei Filterregeln, wobei das zumindest erste Hämatogramm (und gegebenenfalls das zweite Hämatogramm) einen Hämoglobinwert und einen Thrombozytenwert umfasst, und wobei eine Filterregel des ersten Satzes von Filterregeln der ersten Prüfstufe auf dem Hämoglobinwert und dem Thrombozytenwert basiert. Es hat sich gezeigt, dass ein erster Satz von Filterregeln, welcher eine auf dem Hämoglobinwert und dem Thrombozytenwert basierende Filterregel umfasst, vorteilhaft hinsichtlich einer hohen Effektivität und Effizienz der ersten Prüfstufe ist. Diese Filterregel kann beispielsweise als dritte Filterregel bezeichnet werden. Der jeweilige Blutwert kann insbesondere eine Information zur Quantität (beispielsweise eine relative oder absolute Menge, beispielsweise Anzahl/Volumen) des jeweiligen Blutbestandteils umfassen. Gleiches gilt für ein gegebenenfalls zweites Hämatogramm der ersten Prüfstufe.

Gemäß einer bevorzugten Ausgestaltung des Screeningverfahrens gemäß dem ersten Aspekt weicht das zumindest erste Hämatogramm (und gegebenenfalls das zweite Hämatogramm) vom gemäß der auf dem Hämoglobinwert und dem Thrombozytenwert basierenden Filterregel (dritte Filterregel) der ersten Prüfstufe vorgegebenen Sollzustand ab, wenn der Hämoglobinwert unter einem Schwellwert liegt oder diesem entspricht und der Thrombozytenwert unter einem Schwellwert liegt oder diesem entspricht. Es hat sich als besonderes vorteilhaft hinsichtlich der Effektivität und Effizienz des Verfahrens herausgestellt, wenn überprüft wird, ob sowohl Hämoglobinwert unter einem Schwellwert liegt als auch der Thrombozytenwert unter einem Schwellwert liegt. Vorzugsweise ist der Schwellwert für den Hämoglobinwert größer als 8 g/dl. Vorzugsweise ist der Schwellwert für den Hämoglobinwert geringer als 14 g/dl. Vorzugsweise liegt der Schwellwert für den Hämoglobinwert zwischen 10 und 12,5 g/dl. Vorzugsweise ist der Schwellwert für den Thrombozytenwert größer als 80.000, weiter bevorzugt größer als 90.000 Thrombozyten/µL. Vorzugsweise ist der Schwellwert für den Thrombozytenwert geringer als 120.000, weiter vorzugsweise geringer als 110.000 Thrombozyten/µL. Vorzugsweise liegt der Schwellwert für den Thrombozytenwert etwa bei 100.000 Thrombozyten/µL.

Gemäß einer bevorzugten Ausgestaltung des Screeningverfahrens gemäß dem ersten Aspekt, ist der Schwellwert für den Hämoglobinwert ein geschlechtsspezifischer Schwellwert. Hierdurch kann die Effektivität und Effizienz hierauf basierenden Filters (dritte Filterregel) weiter erhöht werden. Mit anderen Worten ist der Schwellwert für den Hämoglobinwert abhängig vom Geschlecht der Person, der die Blutprobe entnommen wurde. Bei weiblichen Personen (oder bei Blutproben, bei denen das Geschlecht der Person nicht bekannt ist) ist der Schwellwert für den Hämoglobinwert vorzugsweise größer als 8 g/dl und/oder vorzugsweise geringer als 12 g/dl, und liegt insbesondere bei etwa 10,6 g/dl. Bei männlichen Personen ist der Schwellwert für den Hämoglobinwert vorzugsweise größer als 10 g/dl und/oder vorzugsweise geringer als 14 g/dl, und liegt insbesondere bei etwa 12,1 g/dl.

Gemäß einem Beispiel des Screeningverfahrens umfasst das zumindest erste Hämatogramm (und gegebenenfalls das zweite Hämatogramm) einen Monozytenwert, wobei eine Filterregel des ersten Satzes von Filterregeln der ersten Prüfstufe auf dem Monozytenwert basiert. Es hat sich gezeigt, dass ein erster Satz von Filterregeln, welcher eine auf dem Monozytenwert basierende Filterregel umfasst, vorteilhaft hinsichtlich einer hohen Effektivität und Effizienz der ersten Prüfstufe ist. Diese Filterregel kann beispielsweise als vierte Filterregel bezeichnet werden. Der Monozytenwert kann insbesondere eine Information zur Quantität (beispielsweise eine relative oder absolute Menge, beispielsweise Anzahl/Volumen) der Monozyten umfassen. Gleiches gilt für ein gegebenenfalls zweites Hämatogramm der ersten Prüfstufe.

Gemäß einem Beispiel des Screeningverfahrens weicht das zumindest erste Hämatogramm (und gegebenenfalls das zweite Hämatogramm) vom gemäß der auf dem Monozytenwert basierenden Filterregel (vierte Filterregel) der ersten Prüfstufe vorgegebenen Sollzustand ab, wenn der Monozytenwert über einem Schwellwert liegt oder diesem entspricht. Vorzugsweise ist der Schwellwert größer als 1.250 Monozyten/µL. Vorzugsweise ist der Schwellwert geringer als 1.750 Monozyten/µL. Vorzugsweise liegt der Schwellwert etwa bei 1.500 Monozyten/µL.

Insbesondere eine Kombination zumindest (vorzugsweise genau) der zuvor beschriebenen vier Filterregeln hat sich als besonders vorteilhaft für eine effektive und effiziente erste Prüfstufe und den Einsatz in Massenscreenings mit einer hohen Anzahl von zu überprüfenden Blutproben erwiesen.

Gemäß einer bevorzugten Ausgestaltung des Screeningverfahrens gemäß dem ersten Aspekt wird im Rahmen der zweiten Prüfstufe ein morphologisches Analyseergebnis einer Festphase der Blutprobe ermittelt und auf Basis eines zweiten Satzes von Filterregeln geprüft, ob das morphologische Analyseergebnis der zweiten Prüfstufe von einem gemäß dem zweiten Satz von Filterregeln vorgegebenen Sollzustand abweicht.

Dadurch, dass die zweite Prüfstufe nur dann durchgeführt wird, wenn die Prüfung der ersten Prüfstufe ergibt, dass eine potentielle hämatologische Neoplasie vorliegt, kann die Anzahl der im Rahmen der zweiten Prüfstufe zu prüfenden Proben deutlich reduziert werden. Wie bereits beschrieben kann durch die erste Prüfstufe erreicht werden, dass etwa nur 1% der ursprünglichen Blutproben der zweiten Prüfstufe unterzogen zu werden brauchen. Dies ermöglicht es, in der zweiten Prüfstufe ein im Vergleich zur ersten Prüfstufe aufwendigeres morphologisches Analyseergebnis einer Festphase der Blutprobe (z.B. basierend auf einem Blutzell-Festphasenträger, welcher etwa durch einen Blutausstrich, durch mit Düsen appliziertem Blut oder anderweitig erzeugt werden kann) zu ermitteln und zur Prüfung heranzuziehen. Durch das Vorsehen einer morphologischen Analyse im Rahmen der zweiten Prüfstufe kann die Effektivität bei gleichzeitig hoher Effizienz des Screeningverfahrens weiter verbessert werden.

Die zweite Prüfstufe wird teilweise oder vollständig automatisiert durchgeführt, beispielsweise durch ein System gemäß dem zweiten Aspekt. Insbesondere kann eine Vorrichtung zur automatischen Anfertigung eines Festphase der Blutprobe und/oder eine Vorrichtung zur morphologischen Analyse (digitaler Zellenmorphologieanalysator) vorgesehen sein. Die Ergebnisse der Vorrichtung zur morphologischen Analyse können jedoch zusätzlich auch händisch nachsortiert bzw. korrigiert werden.

Die Festphase der Blutprobe wird dabei insbesondere mittels Vollblut präpariert. Vorzugsweise ist der so generierte Blutzell-Festphasenträger eingefärbt.

Beispielsweise wird in der zweiten Prüfstufe entschieden, dass eine potentielle hämatologische Neoplasie vorliegt, wenn das morphologische Analyseergebnis der zweiten Prüfstufe von einem gemäß dem zweiten Satz von Filterregeln vorgegebenen Sollzustand abweicht. Dies kann insbesondere der Fall sein, wenn im Rahmen der zweiten Prüfstufe keine weitere Prüfung erfolgt.

Wie bereits in Bezug auf den ersten Satz von Filterregeln ausgeführt, gibt auch der zweite Satz von Filterregeln einen Sollzustand vor, während zudem auch jede Filterregel des zweiten Satzes von Filterregeln für sich jeweils einen Sollzustand vorgibt. So weicht das morphologische Analyseergebnis von einem gemäß dem zweiten Satz von Filterregeln vorgegebenen Sollzustand beispielsweise dann ab, wenn das morphologische Analyseergebnis beispielsweise gemäß lediglich einer einzigen Filterregel, gemäß mehrerer Filterregeln oder aber erst gemäß aller Filterregeln des zweiten Satzes von Filterregeln der zweiten Prüfstufe vom durch die jeweilige(n) Filterregel(n) vorgegebenen Sollzustand abweicht. Wie im Folgenden noch beschrieben wird, ist es allerdings bevorzugt, wenn eine Abweichung bereits gemäß einer einzigen Filterregel des zweiten Satzes von Filterregeln ausreicht, um festzustellen, dass das morphologische Analyseergebnis von einem gemäß dem zweiten Satz von Filterregeln vorgegebenen Sollzustand abweicht.

Ähnlich wie der erste Satz von Filterregeln, umfasst auch der zweite Satz von Filterregeln bevorzugt mehrere Filterregeln. Der zweite Satz von Filterregeln unterscheidet sich jedoch bevorzugt von dem ersten Satz von Filterregeln. Erfindungsgemäß umfasst der zweite Satz von Filterregeln zumindest zwei, vorzugswiese zumindest fünf Filterregeln. Vorzugsweise umfasst der zweite Satz von Filterregeln höchstens 20, vorzugsweise höchstens 15 Filterregeln. Besonders bevorzugt umfasst der zweite Satz von Filterregeln genau zehn Filterregeln. Jede Filterregel kann dabei beispielsweise auf einem oder mehreren Blutwerten basieren.

Gemäß einer bevorzugten Ausgestaltung des Screeningverfahrens gemäß dem ersten Aspekt weicht das morphologische Analyseergebnis der zweiten Prüfstufe von dem gemäß dem zweiten Satz von Filterregeln vorgegebenen Sollzustand bereits dann ab, wenn das morphologische Analyseergebnis der zweiten Prüfstufe von einem gemäß einer Filterregel des zweiten Satzes von Filterregeln vorgegebenen Sollzustand abweicht. Es hat sich gezeigt, dass es auch im Rahmen der zweiten Prüfstufe bei einem Satz von Filterregeln vorteilhaft in Bezug auf ein effektives und effizientes Screening ist, bereits dann einen Abweichung des morphologischen Analyseergebnisses vom durch den gesamten Satz von Filterregeln vorgegebenen Sollzustand anzunehmen, wenn dies gemäß einer Filterregel der Fall ist.

Gemäß einer bevorzugten Ausgestaltung des Screeningverfahrens gemäß dem ersten Aspekt umfasst das morphologische Analyseergebnis der zweiten Prüfstufe einen oder mehrere Zelltypwerte, insbesondere von kernhaltigen Zelltypen, wobei eine oder mehrere Filterregeln des zweiten Satzes von Filterregeln der zweiten Prüfstufe jeweils auf zumindest einem, insbesondere genau einem Zelltypwert basieren.

Ein jeweiliger Zelltypwert kann insbesondere eine Information zur Quantität (beispielsweise eine relative oder absolute Menge) eines jeweiligen Zelltyps sein. Beispielsweise bezieht sich jeder der Filterregeln des zweiten Satzes von Filterregeln auf einen anderen Zelltyp, insbesondere kernhaltigen Zelltyp. Vorzugsweise ist/sind einer oder mehrere (vorzugsweise alle) der Zelltypwerte kernhaltige Zelltypwerte. Vorzugsweise basieren alle Filterregeln des zweiten Satzes von Filterregeln auf zumindest einem, vorzugsweise genau einem Zelltypwert, insbesondere kernhaltigen Zelltypwert.

Gemäß einer bevorzugten Ausgestaltung des Screeningverfahrens gemäß dem ersten Aspekt weicht das morphologische Analyseergebnis der zweiten Prüfstufe vom gemäß einer auf einem Zelltypwert basierenden Filterregel der zweiten Prüfstufe vorgegebenen Sollzustand ab, wenn der jeweilige Zelltypwert über einem jeweiligen Schwellwert liegt oder diesem entspricht. Dabei existiert in der Regel für jeden Zelltypwert ein bestimmter Schwellwert.

Es hat sich gemäß einer bevorzugten Ausgestaltung des Screeningverfahrens gemäß dem ersten Aspekt als vorteilhaft hinsichtlich der Effektivität und Effizienz der zweiten Prüfstufe herausgestellt, wenn einer oder mehrere der Zelltypwerte auf den folgenden Zelltypen basieren:
- Kernschatten;
- vermutlich reaktive atypische Lymphozyten;
- Lymphozyten;
- unklare und/oder neoplastische Lymphozyten;
- Monozyten;
- basophile Granulozyten;
- Myelozyten;
- Promyelozyten;
- Blasten; oder
- Normoblasten.

Vorzugsweise gibt es für jeden Zelltyp zumindest eine, vorzugsweise genau eine Filterregel im zweiten Satz von Filterregeln. Vorzugsweise umfasst der zweite Satz von Filterregeln also zumindest zehn Filterregeln, wobei vorzugsweise jede Filterregel auf jeweils einem Zelltypwert basiert.

Unter dem Zelltyp Kernschatten werden dabei Leukozyten verstanden, die bei der Anfertigung der Festphase der Blutprobe zerquetscht wurden und sich als strukturlose Farbklekse darstellen.

In Bezug auf 100 ausgezählte kernhaltige Zellen sind die jeweiligen Schwellwerte vorzugsweise wie folgt:
Vorzugsweise ist der Schwellwert für den Zelltyp "Kernschatten" (Kernschattenwert) größer als 5, vorzugsweise (zumindest) 10.

Vorzugsweise ist der Schwellwert für den Zelltyp "vermutlich reaktive atypische Lymphozyten" (,vermutlich reaktive atypische Lymphozyten'-Wert) größer als 10, vorzugsweise (zumindest) 15.

Vorzugsweise ist der Schwellwert für den Zelltyp "Lymphozyten" (Lymphozytenwert) größer als 60, vorzugsweise (zumindest) 70.

Der Schwellwert für den Zelltyp "unklare und/oder neoplastische Lymphozyten" (,unklare und/oder neoplastische Lymphozyten'-Wert) ist vorzugsweise (zumindest) 1.

Vorzugsweise ist der Schwellwert für den Zelltyp "Monozyten" (Monozytenwert) größer als 15, vorzugsweise (zumindest) 20.

Der Schwellwert für den Zelltyp "basophile Granulozyten" (,basophile Granulozyten'Wert) ist vorzugsweise (zumindest) 5.

Der Schwellwert für den Zelltyp "Myelozyten" (Myelozytenwert) ist vorzugsweise (zumindest) 5.

Der Schwellwert für den Zelltyp "Promyelozyten" (Promyelozytenwert) ist vorzugsweise (zumindest) 3.

Der Schwellwert für den Zelltyp "Blasten" (Blastenwert) ist vorzugsweise (zumindest) 3.

Der Schwellwert für den Zelltyp "Normoblasten" (Normoblastenwert) ist vorzugsweise (zumindest) 5.

Die oben angegebenen Schwellwerte für die einzelnen Zelltypen haben sich bei der zweiten Prüfstufe (insbesondere in ihrer Kombination) als vorteilhafte Filterregeln erwiesen.

Gemäß einer bevorzugten Ausgestaltung des Screeningverfahrens gemäß dem ersten Aspekt erfolgt im Rahmen der zweiten Prüfstufe eine Prüfung des Plasmas (etwa der Plasmawerte) und/oder des Serums (etwa der Serumwerte) der Blutprobe und/oder des Urins auf hämatologische Neoplasien. Beispielsweise erfolgt im Rahmen der zweiten Prüfstufe immer dann eine Prüfung der Plasmawerte und/oder Serumwerte der Blutprobe und/oder des Urins auf hämatologische Neoplasien, wenn eine Probe mit dem Plasma bzw. Serum bzw. Urin vorliegt.

Erfolgt im Rahmen der zweiten Prüfstufe eine Prüfung des Plasmas und/oder des Serums der Blutprobe und/oder des Urins auf hämatologische Neoplasien, wird beispielsweise in der zweiten Prüfstufe sowohl auf Basis der morphologischen Auswertung als auch auf Basis des Ergebnisses der Prüfung des Plasmas und/oder Serums und/oder des Urins entschieden, ob eine potentielle hämatologische Neoplasie vorliegt.

Gemäß einem zweiten Aspekt der Erfindung wird zudem ein System beschrieben, welches dazu eingerichtet ist oder entsprechende Mittel umfasst, ein Verfahren gemäß dem ersten Aspekt durchzuführen und/oder zu steuern. Das System kann beispielsweise eine oder mehrere Vorrichtungen umfassen. Eine beispielhafte Vorrichtung umfasst etwa eine Datenverarbeitungsanlage, die softwaremäßig und/oder hardwaremäßig eingerichtet ist, um die jeweiligen Schritte eines beispielhaften Verfahrens gemäß dem ersten Aspekt ausführen zu können. Beispiele für eine Datenverarbeitungsanlage sind ein Computer, ein Desktop-Computer, ein Server, ein Thinclient und/oder ein tragbarer Computer. Beispielsweise umfasst das System Vorrichtungen in Form eines ersten und gegebenenfalls eines zweiten automatischen Zellenanalysators für die erste Prüfstufe. Beispielsweise umfasst das System eine Vorrichtung in Form eines digitalen Zellenmorphologieanalysators und/oder eine Vorrichtung zum automatischen Anfertigen der Festphase der Blutprobe für die zweite Prüfstufe.

Zum Beispiel umfasst eine beispielhafte Vorrichtung ferner Mittel zum Speichern von Informationen wie einen Programmspeicher und/oder einen Hauptspeicher. Zum Beispiel umfasst eine beispielhafte erfindungsgemäße Vorrichtung ferner jeweils Mittel zum Empfangen und/oder Senden von Informationen über ein Netzwerk wie eine Netzwerkschnittstelle. Zum Beispiel sind beispielhafte Vorrichtungen über ein oder mehrere Netzwerke miteinander verbunden und/oder verbindbar.

Gemäß einer bevorzugten Ausgestaltung des zweiten Aspekt umfasst das System zumindest einen Prozessor und zumindest einen Speicher mit Computerprogrammcode, wobei der zumindest eine Speicher und der Computerprogrammcode dazu eingerichtet sind, mit dem zumindest einen Prozessor zumindest das Verfahren auszuführen und/oder zu steuern. Unter einem Prozessor soll zum Beispiel eine Kontrolleinheit, ein Mikroprozessor, eine Mikrokontrolleinheit wie ein Mikrocontroller, ein digitaler Signalprozessor (DSP), eine anwendungsspezifische Integrierte Schaltung (ASIC) oder ein Field Programmable Gate Arrays (FPGA) verstanden werden.

Gemäß einem dritten Aspekt der Erfindung wird zudem ein Computerprogrammprodukt beschrieben, umfassend:
- Programmanweisungen, um ein Verfahren gemäß dem ersten Aspekt auszuführen, wenn die Programmanweisungen auf einem Prozessor ausgeführt werden.

Gemäß einem vierten Aspekt der Erfindung wird zudem ein computerlesbares Speichermedium beschrieben, welches ein Computerprogramprodukt gemäß dem dritten Aspekt der Erfindung enthält. Ein beispielhaftes Programm gemäß der Erfindung kann in oder auf einem computerlesbaren Speichermedium gespeichert sein, welches eines oder mehrere beispielhafte Programme enthält und z.B. als magnetisches, elektrisches, elektro-magnetisches, optisches und/oder andersartiges Speichermedium ausgebildet ist. Ein solches computerlesbares Speichermedium ist vorzugsweise gegenständlich (also "berührbar"), zum Beispiel ist es als Datenträgervorrichtung ausgebildet. Eine solche Datenträgervorrichtung ist beispielsweise tragbar oder in einer Vorrichtung fest installiert. Beispiele für eine solche Datenträgervorrichtung sind flüchtige oder nicht-flüchtige Speicher mit wahlfreiem-Zugriff (RAM) wie z.B. NOR-Flash-Speicher oder mit sequentiellen-Zugriff wie NAND-Flash-Speicher und/oder Speicher mit Nur-Lese-Zugriff (ROM) oder Schreib-Lese-Zugriff. Computerlesbar soll zum Beispiel so verstanden werden, dass das Speichermedium von einem Computer bzw. einer Datenverarbeitungsanlage (aus)gelesen und/oder beschrieben werden kann, beispielsweise von einem Prozessor.

In der Zeichnung zeigt
- Fig. 1: ein Diagramm zur schematischen Veranschaulichung des (unentdeckten) Wachstums einer Neoplasie;
- Fig. 2: eine schematische Darstellung eines Ausführungsbeispiels eines Systems gemäß der Erfindung;
- Fig. 3: ein Ablaufdiagramm eines Ausführungsbeispiels eines Verfahrens gemäß der Erfindung; und
- Fig. 4: ein Blockdiagramm einer beispielhaften Vorrichtung eines Ausführungsbeispiels eines Systems gemäß der Erfindung.

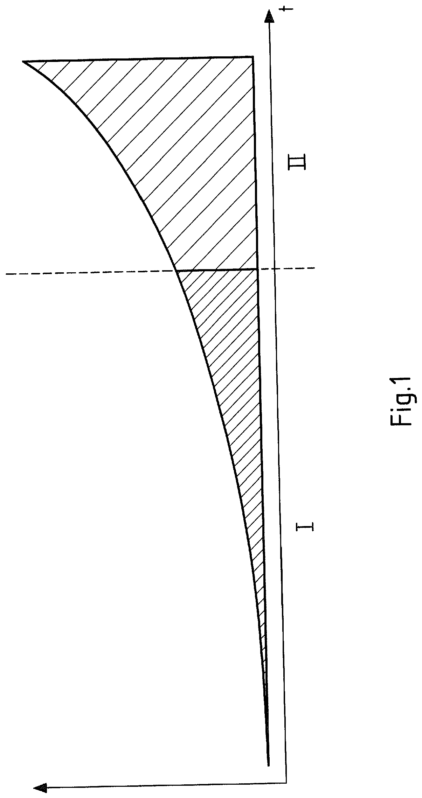

Fig. 1 zeigt zunächst ein Diagramm 100 zur schematischen Veranschaulichung des unentdeckten Wachstums einer Neoplasie, wobei das (klonale) Wachstum bei einer hämatologischen Neoplasie über der Zeit aufgetragen ist. Dabei verläuft das Wachstum zunächst unbemerkt (Phase I) und führt erst im fortgeschrittenen Stadium (Phase II) zu fassbaren Krankheitssymptomen, die sich klinisch manifestieren. Selbst dann sind die verursachten Symptome und Laborbefunde jedoch unspezifisch und von denen weitaus häufigerer Erkrankungen kaum zu unterscheiden. Die Erfindung ermöglicht es nun, sowohl in Phase I als auch in Phase II ein effektives und effizientes Screeningverfahren zur Erkennung von Blutkrebs bereitzustellen.

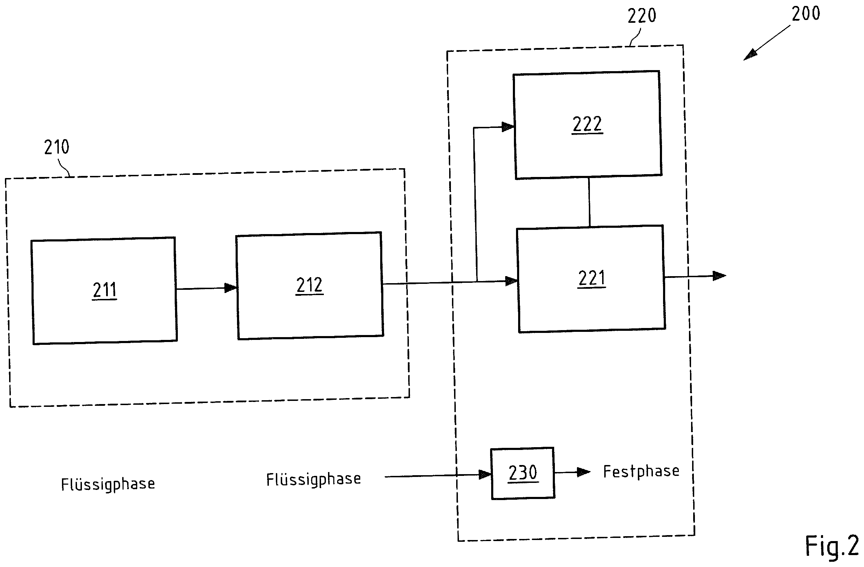

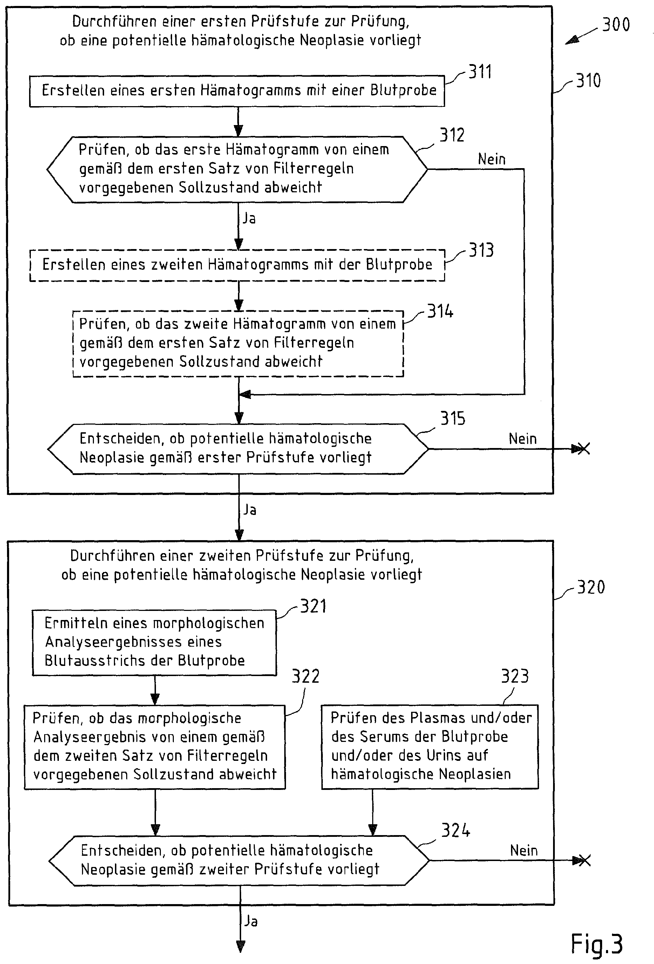

Anhand der Fig. 2 und 3, welche eine schematische Darstellung eines Ausführungsbeispiels eines Systems 200 bzw. eines Verfahrens 300 gemäß der Erfindung, zeigen, soll die Erfindung im Folgenden näher beschrieben werden.

Fig. 2 veranschaulicht zunächst die Einteilung des Systems 200 in zwei Prüfstufen 210 und 220. Die erste Prüfstufe 210 umfasst zwei Zellanalysatoren 211, 212 (erster automatischer Zellenanalysator 211 und zweiter automatischer Zellenanalysator 212) und basiert auf einer Analyse der Blutprobe in der Flüssigphase. Die zweite Prüfstufe 220 umfasst einen digitalen Zellenmorphologieanalysator 221 und basiert auf einer Analyse der Blutprobe in der Festphase, das heißt von Blutzellen, die auf einen Objektträger verbracht wurden z.B. in Form eines Blutausstrichs oder mittels einer Düse. Ein solcher Blutzell-Festphasenträger kann beispielsweise durch Vorrichtung 230 zur automatischen Anfertigung eines Blutzell-Festphasenträgers angefertigt werden. Zudem umfasst die zweite Prüfstufe 220 hier eine Analysevorrichtung 222 zur Prüfung des Plasmas und/oder des Serums der Blutprobe und/oder des Urins.

Zunächst wird mit einer Blutprobe in einer ersten Prüfstufe eine Prüfung durchgeführt, ob eine potentiell hämatologische Neoplasie vorliegt (Aktion 310). Hierzu wird eine Blutprobe einem ersten automatischen Zellenanalysator 211 der ersten Prüfstufe 210 zugeführt. Zunächst wird mit der Blutprobe ein erstes Hämatogramm (beispielsweise ein kleines Blutbild) erstellt (Aktion 311). Anschließend wird geprüft, ob das erste Hämatogramm von einem gemäß einem ersten Satz von Filterregeln vorgegebenen Sollzustand abweicht (Aktion 312).

Der erste Satz von Filterregeln umfasst dabei die folgenden vier Filterregeln:

| | | |
|---|---|---|
| Filterregel 1 | ,Abnormale Lymphozyten'-Wert | > 0 |
| | **ODER** ,unreife Granulozyten'-Wert | > 0 |
| | **ODER** Blastenwert | > 0 |
| Filterregel 2 | Leukozytenwert | > 50.000/µL |
| Filterregel 3 | Thrombozytenwert | < 100.000/µL |
| | **UND** Hämoglobinwert | < 10,6 g/dl (weiblich/unbekannt) |
| | | < 12,1 g/dl (männlich) |
| Filterregel 4 | Monozytenwert | > 1500/µL |

Die obigen Ungleichungen geben in diesem Fall an, wann eine Abweichung gemäß einer Filterregel vorliegt. Dabei wird entschieden, dass das erste Hämatogramm von dem gemäß dem ersten Satz von Filterregeln vorgegebenen Sollzustand abweicht, wenn das erste Hämatogramm bereits von einem gemäß einer einzigen Filterregel der vier Filterregeln vorgegebenen Sollzustand abweicht.

Weicht das erste Hämatogramm nicht ab, kann das Verfahren beendet werden.

Weicht das erste Hämatogramm von dem gemäß dem ersten Satz von Filterregeln vorgegebenen Sollzustand ab, wird mit der Blutprobe weiterhin ein zweites Hämatogramm (beispielsweise ein großes Blutbild) erstellt, welches ein höhere Genauigkeit aufweist als das erste Hämatogramm (Aktion 313). Dies erfolgt mittels des automatischen Zellenanalysators 212 der ersten Prüfstufe 210. Es wird dann erneut auf Basis des ersten Satzes von Filterregeln geprüft, ob auch das zweite Hämatogramm von dem gemäß dem ersten Satz von Filterregeln vorgegebenen Sollzustand abweicht (Aktion 314).

Ist dies nicht der Fall, kann entschieden werden, dass keine potentielle hämatologische Neoplasie vorliegt (Aktion 315). Das Verfahren kann beendet werden. Ist dies jedoch der Fall, kann entschieden werden, dass gemäß der ersten Prüfstufe eine potentielle hämatologische Neoplasie vorliegt (Aktion 315).

Dies hat zur Folge, dass eine zweite Prüfstufe durchgeführt wird, um zu prüfen, ob eine potentielle hämatologische Neoplasie vorliegt (Aktion 320). Im Rahmen der zweiten Prüfstufe müssen aber nur noch etwa 1% der ursprünglichen Proben untersucht werden, da durch die erste Prüfstufe bereits etwa 99% der Proben aussortiert werden konnten.

Die Aktionen 313, 314 können in einer beispielhaften Ausführungsform auch entfallen. In dem Fall wird dann in Aktion 315 lediglich auf Basis der Prüfung des ersten Hämatogramms (Aktion 312) entschieden, ob eine potentielle hämatologische Neoplasie vorliegt.

Hierzu wird ein Blutzell-Festphasenträger mittels einer Vorrichtung 230 zur automatischen Anfertigung eines Blutzell-Festphasenträger angefertigt. Im Rahmen der zweiten Prüfstufe 220 wird dann ein morphologisches Analyseergebnis Festphase der Blutprobe ermittelt (Aktion 321). Es wird auf Basis eines zweiten Satzes von Filterregeln geprüft, ob das morphologische Analyseergebnis der zweiten Prüfstufe von einem gemäß dem zweiten Satz von Filterregeln vorgegebenen Sollzustand abweicht.

Der zweite Satz von Filterregeln umfasst dabei die folgenden zehn Filterregeln, jeweils mit einem Schwellwert in Bezug auf 100 ausgezählte kernhaltige Zellen:

| | | |
|---|---|---|
| Filterregel 5 | Kernschattenwert | > 10 |
| Filterregel 6 | ,vermutlich reaktive atypische Lymphozyten'-Wert | > 15 |
| Filterregel 7 | Lymphozytenwert | > 70 |
| Filterregel 8 | ,unklare und/oder neoplastische Lymphozyten'-Wert | ≥ 1 |
| Filterregel 9 | Monozytenwert | ≥ 20 |
| Filterregel 10 | ,basophile Granulozyten'-Wert | > 5 |
| Filterregel 11 | Myelozytenwert | > 5 |
| Filterregel 12 | Promyelozytenwert | ≥ 3 |
| Filterregel 13 | Blastenwert | ≥ 3 |
| Filterregel 14 | Normoblastenwert | ≥ 5 |

Die obigen Ungleichungen geben auch in diesem Fall an, wann eine Abweichung gemäß einer Filterregel vorliegt. Es wird dann geprüft, ob das morphologische Analyseergebnis von dem gemäß dem zweiten Satz von Filterregeln vorgegebenen Sollzustand abweicht (Aktion 322). Dies ist der Fall, wenn das morphologische Analyseergebnis bereits von einem gemäß einer einzigen Filterregel der zehn Filterregeln vorgegebenen Sollzustand abweicht.

Weicht das morphologische Analyseergebnis von dem vorgegebenen Sollzustand nicht ab, kann entschieden werden, dass keine potentiell hämatologische Neoplasie vorliegt (Aktion 324). Das Verfahren kann in dem Fall beendet werden. Weicht das morphologische Analyseergebnis allerdings von dem vorgegebenen Sollzustand ab, kann in Aktion 324 entschieden werden, dass eine potentielle hämatologische Neoplasie vorliegt.

Optional kann zudem im Rahmen der zweiten Prüfstufe 220 mittels einer Analysevorrichtung 222 weiterhin eine Prüfung des Plasmas und/oder des Serums der Blutprobe und/oder des Urins auf hämatologische Neoplasien erfolgen (Aktion 323), sofern eine entsprechende Probe verfügbar ist. Das Ergebnis der Untersuchung des Plasmas und/oder Serums und/oder des Urins kann beispielsweise die Entscheidung in Aktion 324 beeinflussen, ob eine potentiell hämatologische Neoplasie vorliegt. Alternativ kann das Ergebnis auch lediglich für weitere nachgelagerte Verfahrensschritte zur Verfügung gestellt werden.

Wird in Aktion 324 entschieden, dass keine potentiell hämatologische Neoplasie vorliegt, kann das Verfahren beendet werden. Wird in Aktion 324 hingegen entschieden, dass eine potentiell hämatologische Neoplasie vorliegt, können weitere Verfahrensschritte, beispielsweise weitere manuelle oder automatisierte Analysen, durchgeführt werden, um einen abschließenden Befund zu erstellen.

Allerdings müssen die weiteren Verfahrensschritte nur noch für rund 2% der die zweite Prüfstufe durchlaufenden Proben durchgeführt werden, das heißt nur noch für rund 0,02% der ursprünglich zu untersuchenden Proben.

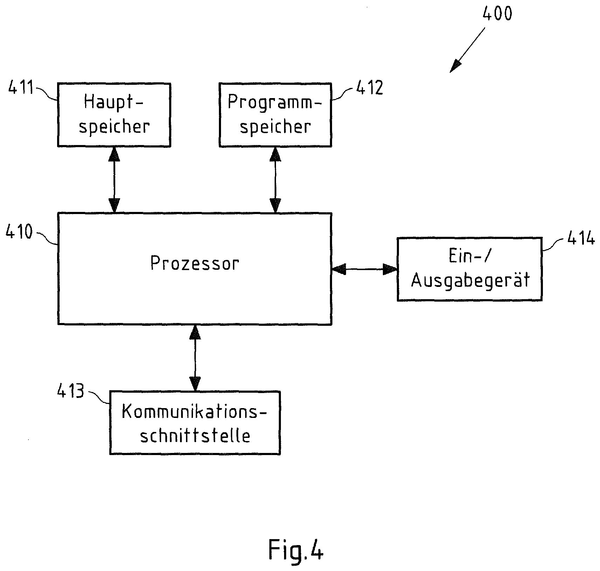

Fig. 4 zeigt schließlich ein Blockdiagramm einer beispielhaften Vorrichtung 400, wie sie beispielsweise in dem System 200, beispielsweise als Vorrichtung 211, 212, 221, 222 und/oder 230 zum Einsatz kommen kann.

Die Vorrichtung 400 kann insofern beispielsweise eine Datenverarbeitungsanlage sein oder umfassen.

Prozessor 410 der Vorrichtung 400 ist insbesondere als Mikroprozessor, Mikrokontrolleinheit, Mikrocontroller, digitaler Signalprozessor (DSP), Anwendungsspezifische Integrierte Schaltung (ASIC) oder Field Programmable Gate Array (FPGA) ausgebildet.

Prozessor 410 führt Programmanweisungen aus, die in Programmspeicher 412 gespeichert sind, und speichert beispielsweise Zwischenergebnisse oder ähnliches in Arbeits- oder Hauptspeicher 411. Zum Beispiel ist Programmspeicher 412 ein nicht-flüchtiger Speicher wie ein Flash-Speicher, ein Magnetspeicher, ein EEPROM-Speicher (elektrisch löschbarer programmierbarer Nur-Lese-Speicher) und/oder ein optischer Speicher. Hauptspeicher 411 ist zum Beispiel ein flüchtiger oder nicht-flüchtiger Speicher, insbesondere ein Speicher mit wahlfreiem-Zugriff (RAM) wie ein statischer RAM-Speicher (SRAM), ein dynamischer RAM-Speicher (DRAM), ein ferroelektrischer RAM-Speicher (FeRAM) und/oder ein magnetischer RAM-Speicher (MRAM).

Programmspeicher 412 ist vorzugsweise ein lokaler mit der Vorrichtung 400 fest verbundener Datenträger. Mit der Vorrichtung 400 fest verbundene Datenträger sind beispielsweise Festplatten, die in die Vorrichtung 400 eingebaut sind. Alternativ kann der Datenträger beispielsweise auch ein mit der Vorrichtung 400 trennbar verbindbarer Datenträger sein wie ein Speicher-Stick, ein Wechseldatenträger, eine tragbare Festplatte, eine CD, eine DVD und/oder eine Diskette.

Programmspeicher 412 enthält beispielsweise das Betriebssystem von der Vorrichtung 400, das beim Starten der Vorrichtung 400 zumindest teilweise in Hauptspeicher 411 geladen und vom Prozessor 410 ausgeführt wird. Insbesondere wird beim Starten von Vorrichtung 400 zumindest ein Teil des Kerns des Betriebssystems in den Hauptspeicher 411 geladen und von Prozessor 410 ausgeführt.

Das Betriebssystem ermöglicht insbesondere die Verwendung der Vorrichtung 400 zur Datenverarbeitung. Es verwaltet beispielsweise Betriebsmittel wie Hauptspeicher 411 und Programmspeicher 412, Netzwerkschnittstelle 413, Ein- und Ausgabegerät 414, stellt unter anderem durch Programmierschnittstellen anderen Programmen grundlegende Funktionen zur Verfügung und steuert die Ausführung von Programmen.

Prozessor 410 steuert die Kommunikationsschnittstelle 413, welche beispielsweise eine Netzwerkschnittstelle sein kann und als Netzwerkkarte, Netzwerkmodul und/oder Modem ausgebildet sein kann. Die Kommunikationsschnittstelle 413 ist insbesondere dazu eingerichtet, eine Verbindung der Vorrichtung 400 mit anderen Vorrichtungen, insbesondere über ein (drahtloses) Kommunikationssystem, beispielsweise ein Netzwerk, herzustellen und mit diesen zu kommunizieren, beispielsweise anderen Vorrichtungen des Systems 200. Die Kommunikationsschnittstelle 413 kann beispielsweise Daten (über das Kommunikationssystem) empfangen und an Prozessor 410 weiterleiten und/oder Daten von Prozessor 410 empfangen und (über das Kommunikationssystem) senden. Beispiele für ein Kommunikationssystem sind ein lokales Netzwerk (LAN), ein großräumiges Netzwerk (WAN), ein drahtloses Netzwerk (beispielsweise gemäß dem IEEE-802.11-Standard, dem Bluetooth (LE)-Standard und/oder dem NFC-Standard), ein drahtgebundenes Netzwerk, ein Mobilfunknetzwerk, ein Telefonnetzwerk und/oder das Internet.

Des Weiteren kann Prozessor 410 zumindest ein Ein-/Ausgabegerät 414 steuern. Ein-/Ausgabegerät 414 ist beispielsweise eine Tastatur, eine Maus, eine Anzeigeeinheit, ein Mikrofon, eine berührungsempfindliche Anzeigeeinheit, ein Lautsprecher, ein Lesegerät, ein Laufwerk und/oder eine Kamera. Ein-/Ausgabegerät 414 kann beispielsweise Eingaben eines Benutzers aufnehmen und an Prozessor 410 weiterleiten und/oder Informationen für den Benutzer von Prozessor 410 empfangen und ausgeben.

Schließlich kann eine entsprechende Vorrichtung auch weitere Mittel aufweisen, die für Verfahrensschritte im Rahmen des erfindungsgemäßen Verfahrens verwendet werden können, wie beispielsweise Analyseeinheiten zur automatisierten Erstellung eines Hämatogramms oder einer digitalen morphologischen Analyse.

## Patentansprüche

1. Screeningverfahren zur Diagnose einer hämatologischen Neoplasie, umfassend die Schritte:
- Durchführen einer ersten Prüfstufe (210) zur Prüfung, ob eine potentielle hämatologische Neoplasie vorliegt,
wobei die erste Prüfstufe (210) teilweise oder vollständig automatisiert durchgeführt wird,
wobei im Rahmen der ersten Prüfstufe (210) zumindest ein erstes Hämatogramm mit einer Blutprobe in der Flüssigphase erstellt wird und auf Basis eines ersten Satzes von Filterregeln geprüft wird, ob das erste Hämatogramm von einem gemäß dem ersten Satz von Filterregeln vorgegebenen Sollzustand abweicht,
wobei der erste Satz von Filterregeln zumindest zwei Filterregeln umfasst,
wobei das zumindest erste Hämatogramm einen Hämoglobinwert und einen Thrombozytenwert umfasst, und
wobei eine Filterregel des ersten Satzes von Filterregeln der ersten Prüfstufe (210) auf dem Hämoglobinwert und dem Thrombozytenwert basiert, und
- Durchführen einer zweiten Prüfstufe (220) zur Prüfung, ob eine potentielle hämatologische Neoplasie vorliegt, wenn die Prüfung der ersten Prüfstufe (210) ergibt, dass eine potentielle hämatologische Neoplasie vorliegt,
wobei die zweite Prüfstufe (220) teilweise oder vollständig automatisiert durchgeführt wird,
wobei im Rahmen der zweiten Prüfstufe (220) ein morphologisches Analyseergebnis einer Festphase der Blutprobe ermittelt wird und auf Basis eines zweiten Satzes von Filterregeln geprüft wird, ob das morphologische Analyseergebnis der zweiten Prüfstufe (220) von einem gemäß dem zweiten Satz von Filterregeln vorgegebenen Sollzustand abweicht, und
wobei der zweite Satz von Filterregeln zumindest zwei Filterregeln umfasst.

2. Screeningverfahren nach Anspruch 1,
wobei im Rahmen der ersten Prüfstufe (210), wenn das erste Hämatogramm von dem gemäß dem ersten Satz von Filterregeln vorgegebenen Sollzustand abweicht, mit der Blutprobe weiterhin ein zweites Hämatogramm erstellt wird, welches ein höhere Genauigkeit aufweist als das erste Hämatogramm, und auf Basis des ersten Satzes von Filterregeln geprüft wird, ob das zweite Hämatogramm von dem gemäß dem ersten Satz von Filterregeln vorgegebenen Sollzustand abweicht.

3. Screeningverfahren nach Anspruch 1 oder 2,
wobei das zumindest erste Hämatogramm von dem gemäß dem ersten Satz von Filterregeln vorgegebenen Sollzustand bereits dann abweicht, wenn das zumindest erste Hämatogramm von einem gemäß einer Filterregel des ersten Satzes von Filterregeln vorgegebenen Sollzustand abweicht.

4. Screeningverfahren nach einem der vorhergehenden Ansprüche,
wobei das zumindest erste Hämatogramm einen ,abnormale Lymphozyten'-Wert, einen ,unreife Granulozyten'-Wert und/oder einen Blastenwert umfasst, und
wobei eine Filterregel des ersten Satzes von Filterregeln der ersten Prüfstufe (210) auf dem ,abnormale Lymphozyten'-Wert, dem ,unreife Granulozyten'-Wert und/oder dem Blastenwert basiert.

5. Screeningverfahren nach einem der vorhergehenden Ansprüche,
wobei das zumindest erste Hämatogramm einen Leukozytenwert umfasst, und
wobei eine Filterregel des ersten Satzes von Filterregeln der ersten Prüfstufe (210) auf dem Leukozytenwert basiert.

6. Screeningverfahren nach Anspruch 5,
wobei das zumindest erste Hämatogramm vom gemäß der auf dem Leukozytenwert basierenden Filterregel der ersten Prüfstufe (210) vorgegebenen Sollzustand abweicht, wenn der Leukozytenwert über einem Schwellwert liegt oder diesem entspricht.

7. Screeningverfahren nach einem der Ansprüche 1 bis 6,
wobei das zumindest erste Hämatogramm vom gemäß der auf dem Hämoglobinwert und dem Thrombozytenwert basierenden Filterregel der ersten Prüfstufe (210) vorgegebenen Sollzustand abweicht, wenn der Hämoglobinwert unter einem Schwellwert liegt oder diesem entspricht und der Thrombozytenwert unter einem Schwellwert liegt oder diesem entspricht.

8. Screeningverfahren nach Anspruch 7,
wobei der Schwellwert für den Hämoglobinwert ein geschlechtsspezifischer Schwellwert ist.

9. Screeningverfahren nach einem der vorhergehenden Ansprüche,
wobei das zumindest erste Hämatogramm einen Monozytenwert umfasst, und
wobei eine Filterregel des ersten Satzes von Filterregeln der ersten Prüfstufe (210) auf dem Monozytenwert basiert.

10. Screeningverfahren nach Anspruch 9,
wobei das zumindest erste Hämatogramm vom gemäß der auf dem Monozytenwert basierenden Filterregel der ersten Prüfstufe (210) vorgegebenen Sollzustand abweicht, wenn der Monozytenwert über einem Schwellwert liegt oder diesem entspricht.

11. Screeningverfahren nach einem der Ansprüche 1 bis 10,
wobei das morphologische Analyseergebnis der zweiten Prüfstufe (220) von dem gemäß dem zweiten Satz von Filterregeln vorgegebenen Sollzustand bereits dann abweicht, wenn das morphologische Analyseergebnis der zweiten Prüfstufe (220) von einem gemäß einer Filterregel des zweiten Satzes von Filterregeln vorgegebenen Sollzustand abweicht.

12. Screeningverfahren nach einem der Ansprüche 1 bis 11,
wobei das morphologische Analyseergebnis der zweiten Prüfstufe (220) einen oder mehrere Zelltypwerte, insbesondere von kernhaltigen Zelltypen, umfasst, und wobei eine oder mehrere Filterregeln des zweiten Satzes von Filterregeln der zweiten Prüfstufe (220) jeweils auf zumindest einem, insbesondere genau einem Zelltypwert basieren.

13. Screeningverfahren nach Anspruch 12,
wobei das morphologische Analyseergebnis der zweiten Prüfstufe (220) vom gemäß einer auf einem Zelltypwert basierenden Filterregel der zweiten Prüfstufe (220) vorgegebenen Sollzustand abweicht, wenn der jeweilige Zelltypwert über einem jeweiligen Schwellwert liegt oder diesem entspricht.

14. Screeningverfahren nach Anspruch 12 oder 143,
wobei einer oder mehrere der Zelltypwerte auf den folgenden Zelltypen basieren:
- Kernschatten;
- vermutlich reaktive atypische Lymphozyten;
- Lymphozyten;
- unklare und/oder neoplastische Lymphozyten;
- Monozyten;
- basophile Granulozyten;
- Myelozyten;
- Promyelozyten;
- Blasten; oder
- Normoblasten.

15. Screeningverfahren nach einem der vorhergehenden Ansprüche,
wobei im Rahmen der zweiten Prüfstufe (220) eine Prüfung des Plasmas und/oder des Serums der Blutprobe und/oder des Urins auf hämatologische Neoplasien erfolgt.

16. System (200), welches dazu eingerichtet ist oder entsprechende Mittel umfasst, ein Verfahren nach einem der Ansprüche 1 bis 15 durchzuführen und/oder zu steuern.

17. System (200) nach Anspruch 16, wobei das System zumindest einen Prozessor (410) und zumindest einen Speicher (411, 412) mit Computerprogrammcode umfasst, wobei der zumindest eine Speicher (411, 412) und der Computerprogrammcode dazu eingerichtet sind, mit dem zumindest einen Prozessor (410) zumindest das Verfahren auszuführen und/oder zu steuern.

18. Computerprogrammprodukt umfassend:
- Programmanweisungen, um ein Verfahren nach einem der Ansprüche 1 bis 15 auszuführen, wenn die Programmanweisungen auf einem Prozessor (410) ausgeführt werden.

19. Computerlesbares Speichermedium, welches ein Computerprogramprodukt gemäß Anspruch 18 enthält.

## Claims

1. Screening method for diagnosis of haematological neoplasia, comprising the steps of:
- performing a first test step (210) to check for potential haematological neoplasia,
wherein the first test step (210) is performed partially or fully automated,
wherein as part of the first test step (210) at least a first haematogram is created with a blood sample in the liquid phase and it is checked based on a first set of filter rules whether the first haematogram deviates from a desired state specified according to the first set of filter rules, and
wherein the first set of filter rules comprises at least two filter rules,
wherein the at least first haematogram comprises a haemoglobin value and a platelets value, and
wherein a filter rule of the first set of filter rules of the first test step (210) is based on the haemoglobin value and the platelets value, and
- performing a second test step (220) to check for potential haematological neoplasia if the first test step (210) indicates that there is potential haematological neoplasia,
wherein the second test step (220) is partially or completely automated,
wherein as part of the second test step (220) a morphological analysis result of a solid phase of the blood sample is determined and it is checked based on a second set of filter rules whether the morphological analysis result of the second test step (220) deviates from a desired state specified according to the second set of filter rules, and
wherein the second set of filter rules comprises at least two filter rules.

2. Screening method according to claim 1,
wherein as part of the first test step (210), if the first haematogram deviates from the desired state specified according to the first set of filter rules, a second haematogram, which has a higher accuracy than the first, is created with the blood sample, and based on the first set of filter rules it is checked whether the second haematogram deviates from the desired state specified according to the first set of filter rules.

3. Screening method according to claim 1 or claim 2,
wherein the at least first haematogram deviates from the desired state specified according to the first set of filter rules already when the at least first haematogram deviates from a desired state specified according to a filter rule of the first set of filter rules.

4. Screening method according to any one of the preceding claims,
wherein the at least first haematogram comprises an 'abnormal lymphocytes' value, an 'immature granulocytes' value and/or a blast value, and wherein a filter rule of the first set of filter rules of the first test step (210) is based on the 'abnormal lymphocytes' value, the 'immature granulocytes' value and/or the blast value.

5. Screening method according to any one of the preceding claims,
wherein the at least first haematogram comprises a leucocytes value, and
wherein a filter rule of the first set of filter rules of the first test step (210) is based on the leucocytes value.

6. Screening method according to claim 5,
wherein the at least first haematogram deviates from the desired state specified according to the filter rule of the first test step (210) based on the leucocytes value when the leucocytes value is above a threshold or corresponds to this.

7. Screening method according to any of claims 1 to 6,
wherein the at least first haematogram deviates from the desired state specified according to the filter rule based on the haemoglobin value and platelets value of the first test step (210), if the haemoglobin value is below a threshold or corresponds to this and the platelets value is below or corresponds to a threshold value.

8. Screening method according to claim 7,
wherein the threshold for the haemoglobin value is a gender-specific threshold.

9. Screening method according to any one of the preceding claims,
wherein the at least first haematogram comprises a monocytes value, and wherein a filter rule of the first set of filter rules of the first test step (210) is based on the monocytes value.

10. Screening method according to claim 9,
wherein the at least first haematogram deviates from the desired state specified according to the filter rule of the first test step (210) based on the monocytes value, when the monocytes value is above a threshold or corresponds to this.

11. Screening method according to any one of claims 1 to 10,
wherein the morphological analysis result of the second test step (220) already deviates from the desired state specified according to the second set of filter rules if the morphological analysis result of the second test step (220) deviates from a desired state specified according to a filter rule of the second set of filter rules.

12. Screening method according to any one of claims 1 to 11,
wherein the morphological analysis result of the second test step (220) comprises one or more cell type values, particularly of nucleated cell types, and
wherein one or more filter rules of the second set of filter rules of the second test step (220) are each based on at least one, in particular, exactly one cell type value.

13. Screening method according to claim 12,
wherein the morphological analysis result of the second test step (220) deviates from the desired state specified by a filter rule based on a cell-type value of the second test step (220) if the respective cell-type value is greater than or equal to a respective threshold.

14. Screening method according to claim 12 or claim 13,
wherein one or more of the cell type values are based on the following cell types:
- shadow cells;
- likely reactive atypical lymphocytes;
- lymphocytes;
- unclear and/or neoplastic lymphocytes;
- monocytes;
- basophilic granulocytes;
- myelocytes;
- promyelocytes;
- blasts; or
- normoblasts.

15. Screening method according to any one of the preceding claims,
wherein the second test step (220) involves testing the plasma and/or serum of the blood sample and/or urine for haematological neoplasia.

16. System (200) which is configured or comprises corresponding means for carrying out and/or controlling a method according to any one of claims 1 to 15.

17. System (200) according to claim 16, wherein the system comprises at least one processor (410) and at least one memory (411, 412) with computer program code, wherein the at least one memory (411, 412) and the computer program code are configured to at least execute and/or control the process with the at least one processor (410).

18. Computer program product comprising:
- program instructions for carrying out a method according to any one of claims 1 to 15 when the program instructions are executed on a processor (410).

19. Computer readable storage medium containing a computer program product according to claim 18.

## Revendications

1. Procédé de dépistage destiné au diagnostic d'un néoplasme hématologique, comprenant les étapes dans lesquelles :
- on met en œuvre une première étape de contrôle (210) afin de vérifier la présence ou non d'un néoplasme hématologique potentiel ;
dans lequel la première étape de contrôle (210) est mise en œuvre en partie ou totalement de manière automatisée ;
dans lequel, dans le cadre de la première étape de contrôle (210), on établit au moins un premier hémogramme avec un échantillon de sang dans la phase liquide et, sur base d'une première série de règles de filtrage, on vérifie le fait de savoir si le premier hémogramme s'écarte ou non d'un état de consigne prédéfini en conformité avec la première série de règles de filtrage ;
dans lequel la première série de règles de filtrage comprend au moins deux règles de filtrage ;
dans lequel ledit au moins un premier hémogramme comprend un taux d'hémoglobine et un taux de thrombocytes ; et
dans lequel une règle de filtrage de la première série de règles de filtrage de la première étape de contrôle (210) se base sur le taux d'hémoglobine et sur le taux de thrombocytes ; et
- on met en œuvre une deuxième étape de contrôle (220) afin de vérifier la présence ou non d'un néoplasme hématologique potentiel lorsque la vérification de la première étape de contrôle (210) laisse apparaître la présence d'un néoplasme hématologique potentiel ;
dans lequel la deuxième étape de contrôle (220) est mise en œuvre en partie ou totalement de manière automatisée ;
dans lequel, dans le cadre de la deuxième étape de contrôle (220), on détermine un résultat de l'analyse morphologique d'une phase solide de l'échantillon de sang, et sur base d'une deuxième série de règles de filtrage, on vérifie le fait de savoir si le résultat de l'analyse morphologique de la deuxième étape de contrôle (220) s'écarte ou non d'un état de consigne prédéfini en conformité avec la deuxième série de règles de filtrage ; et
dans lequel la deuxième série de règles de filtrage comprend au moins deux règles de filtrage.

2. Procédé de dépistage selon la revendication 1,
dans lequel, dans le cadre de la première étape de contrôle (210), lorsque le premier hémogramme s'écarte de l'état de consigne prédéfini en conformité avec la première série de règles de filtrage, on établit, avec l'échantillon de sang, en outre un deuxième hémogramme qui présente une précision supérieure à celle du premier hémogramme, et, sur base de la première série de règles de filtrage, on vérifie le fait de savoir si le deuxième hémogramme s'écarte ou non de l'état de consigne prédéfini en conformité avec la première série de règles de filtrage.

3. Procédé de dépistage selon la revendication 1 ou 2,
dans lequel ledit au moins un premier hémogramme s'écarte déjà de l'état de consigne prédéfini en conformité avec la première série de règles de filtrage lorsque ledit au moins un premier hémogramme s'écarte d'un état de consigne prédéfini en conformité avec une règle de filtrage de la première série de règles de filtrage.

4. Procédé de dépistage selon l'une quelconque des revendications précédentes,
dans lequel ledit au moins un premier hémogramme comprend un taux de « lymphocytes anormaux », un taux de « granulocytes immatures » et/ou un taux de blastes; et dans lequel une règle de filtrage de la première série de règles de filtrage de la première étape de contrôle (210) se base sur le taux de « lymphocytes anormaux », sur le taux de « granulocytes immatures » et/ou sur le taux de blastes.

5. Procédé de dépistage selon l'une quelconque des revendications précédentes,
dans lequel ledit au moins un premier hémogramme comprend un taux de leucocytes ; et dans lequel une règle de filtrage de la première série de règles de filtrage de la première étape de contrôle (210) se base sur le taux de leucocytes.

6. Procédé de dépistage selon la revendication 5,
dans lequel ledit au moins un premier hémogramme s'écarte de l'état de consigne prédéfini en conformité avec la règle de filtrage de la première étape de contrôle (210), qui se base sur le taux de leucocytes, lorsque le taux de leucocytes est supérieur à une valeur seuil ou correspond à cette dernière.

7. Procédé de dépistage selon l'une quelconque des revendications 1 à 6, dans lequel ledit au moins un premier hémogramme s'écarte de l'état de consigne prédéfini en conformité avec la règle de filtrage de la première étape de contrôle (210), qui se base sur le taux d'hémoglobine et sur le taux de thrombocytes, lorsque le taux d'hémoglobine est inférieur à une valeur seuil ou correspond à cette dernière et lorsque le taux de thrombocytes est inférieur à une valeur seuil ou correspond à cette dernière.

8. Procédé de dépistage selon la revendication 7,
dans lequel la valeur seuil pour le taux d'hémoglobine représente une valeur seuil sexospécifique.

9. Procédé de dépistage selon l'une quelconque des revendications précédentes,
dans lequel ledit au moins un premier hémogramme comprend un taux de monocytes ; et dans lequel une règle de filtrage de la première série de règles de filtrage de la première étape de contrôle (210) se base sur le taux de monocytes.

10. Procédé de dépistage selon la revendication 9,
dans lequel ledit au moins un premier hémogramme s'écarte de l'état de consigne prédéfini en conformité avec la règle de filtrage de la première étape de contrôle (210), qui se base sur le taux de monocytes, lorsque le taux de monocytes est supérieur à une valeur seuil ou correspond à cette dernière.

11. Procédé de dépistage selon l'une quelconque des revendications 1 à 10, dans lequel le résultat de l'analyse morphologique de la deuxième étape de contrôle (220) s'écarte de l'état de consigne prédéfini en conformité avec la deuxième série de règles de filtrage, déjà lorsque le résultat de l'analyse morphologique de la deuxième étape de contrôle (220) s'écarte d'un état de consigne prédéfini en conformité avec une règle de filtrage de la deuxième série de règles de filtrage.

12. Procédé de dépistage selon l'une quelconque des revendications 1 à 11, dans lequel le résultat de l'analyse morphologique de la deuxième étape de contrôle (220) comprend un ou plusieurs taux de types de cellules, en particulier des types de cellules nucléées ; et dans lequel une ou plusieurs règles de filtrage de la deuxième série de règles de filtrage de la deuxième étape de contrôle (220) se basent respectivement sur au moins un taux de type de cellule, en particulier sur précisément un taux de type de cellule.

13. Procédé de dépistage selon la revendication 12,
dans lequel le résultat de l'analyse morphologique de la deuxième étape de contrôle (220) s'écarte de l'état de consigne prédéfini en conformité avec une règle de filtrage de la deuxième étape de contrôle (220), qui se base sur un taux de type de cellule, lorsque le taux de type de cellule respectif est supérieur à une valeur seuil respective ou correspond à cette dernière.

14. Procédé de dépistage selon la revendication 12 ou 13,
dans lequel un ou plusieurs taux de types de cellules se basent sur les types de cellules suivants :
- des ombres nucléaires ;
- des lymphocytes atypiques vraisemblablement réactifs ;
- des lymphocytes ;
- des lymphocytes imprécis et/ou néoplasiques ;
- des monocytes ;
- des granulocytes basophiles ;
- des myélocytes ;
- des promyélocytes ;
- des blastes ; ou
- des normoblastes.

15. Procédé de dépistage selon l'une quelconque des revendications précédentes,
dans lequel, dans le cadre de la deuxième étape de contrôle (220), a lieu une vérification du plasma et/ou du sérum de l'échantillon de sang et/ou de l'urine en ce qui concerne la présence de néoplasmes hématologiques.

16. Système (200) qui est conçu pour ou qui comprend des moyens correspondants pour la mise en œuvre et/ou pour la commande d'un procédé selon l'une quelconque des revendications 1 à 15.

17. Système (200) selon la revendication 16, dans lequel le système comprend au moins un processeur (410) et au moins une mémoire (411, 412) comprenant un code de programme informatique; dans lequel ladite au moins une mémoire (411, 412) et le code de programme informatique sont conçus pour au moins mettre en œuvre et/ou commander le procédé avec ledit au moins un processeur (410).

18. Produit de programme informatique comprenant :
- des instructions de programme destinées à la mise en œuvre d'un procédé selon l'une quelconque des revendications 1 à 15, lorsque les instructions de programme sont mises en œuvre sur un processeur (410).

19. Support de stockage lisible par ordinateur, qui contient un produit de programme informatique selon la revendication 18.
